# EUROPEAN PATENT APPLICATION

(11) **EP 2 140 866 A1**
(43) Date of publication of application: **06.01.2010**
(21) Application number: 08012074.4
(22) Date of filing: 04.07.2008
(51) Int. Cl.: A61K 31/422, A61K 31/454, A61K 31/5355, A61K 31/5377, A61P 1/00, A61P 9/10

(54) **Oxazolidinones for the treatment of inflammatory conditions of the gastrointestinal tract**

(71) Applicant: Bayer Schering Pharma Aktiengesellschaft, 13353 Berlin (DE)
(72) Inventor: Degenfeld, Georges Dr., 51373 Leverkusen (DE); Perzborn, Elisabeth Dr., 42327 Wuppertal (DE); Thielemann, Wolfgang Dr., 42107 Wuppertal (DE); Röhrig, Susanne Dr., 40724 Hilden (DE); McDonald, Fiona, 14055 Berlin (DE)

(57) **Abstract**

The present invention relates to the use of selective inhibitors of coagulation factor Xa, in particular of oxazolidinones of the formula (I), for the treatment and/or prophylaxis of inflammatory conditions of the gastrointestinal tract such as inflammatory bowel disease (IBD) and celiac disease and/or disorders related to inflammatory bowel disease (IBD) and/or celiac disease such as thromboembolic events (including e.g. pulmonary embolism (PE), arterial and venous thrombosis, myocardial infarction, stroke) as well as their use for the preparation of pharmaceutical drugs for the treatment and/or prophylaxis of inflammatory bowel disease (IBD) and/or celiac disease and/or disorders related to inflammatory bowel disease (IBD) and/or celiac disease such as thromboembolic events.

## Description

The present invention relates to the use of selective inhibitors of coagulation factor Xa, in particular of oxazolidinones of the formula (I), for the treatment and/or prophylaxis of inflammatory conditions of the gastrointestinal tract such as inflammatory bowel disease (IBD) and celiac disease and/or disorders related to inflammatory bowel disease (IBD) and/or celiac disease such as thromboembolic events (including e.g. pulmonary embolism (PE), arterial and venous thrombosis, myocardial infarction, stroke) as well as their use for the preparation of pharmaceutical drugs for the treatment and/or prophylaxis of inflammatory bowel disease (IBD) and/or celiac disease and/or disorders related to inflammatory bowel disease (IBD) and/or celiac disease such as thromboembolic events.

Oxazolidinones of the formula (I) are known from WO-A-01/047919 and act in particular as selective inhibitors of coagulation factor Xa and as anticoagulants.

Oxazolidinones of the formula (I) inhibit the coagulation factor Xa selectively. It has been possible to demonstrate an antithrombotic effect of factor Xa inhibitors in numerous animal models (cf. U. Sinha, P. Ku, J. Malinowski, B. Yan Zhu, R.M. Scarborough, C.K. Marlowe, P.W. Wong, P. Hua Lin, S.J. Hollenbach, Antithrombotic and hemostatic capacity of factor Xa versus thrombin inhibitors in models of venous and arteriovenous thrombosis, European Journal of Pharmacology 2000, 395, 51-59; A. Betz, Recent advances in Factor Xa inhibitors, Expert Opin. Ther. Patents 2001, 11, 1007; K. Tsong Tan, A. Makin, G.Y.H. Lip, Factor X inhibitors, Exp. Opin. Investig. Drugs 2003, 12, 799; J. Ruef, H.A. Katus, New antithrombotic drugs on the horizon, Expert Opin. Investig. Drugs 2003, 12, 781; M.M. Samama, Synthetic direct and indirect factor Xa inhibitors, Thrombosis Research 2002, 106, V267; M.L. Quan, J.M. Smallheer, The race to an orally active Factor Xa inhibitor, Recent advances, J. Current Opinion in Drug Discovery & Development 2004, 7, 460-469) and in clinical studies on patients (The Ephesus Study, Blood 2000, 96, 490a; The Penthifra Study, Blood 2000, 96, 490a; The Pentamaks Study, Blood 2000, 96, 490a-491a; The Pentathlon Study, Blood 2000, Vol 96, 491a). Factor Xa inhibitors can therefore be employed preferably in medicaments for the prophylaxis and/or treatment of thromboembolic disorders.

Selective factor Xa inhibitors show a broad therapeutic window. It has been possible to demonstrate in numerous animal models that selective factor Xa inhibitors show an antithrombotic effect without or only marginally prolongating the bleeding time (cf. RJ Leadly, Coagulationfactor Xa inhibition: biological background and rationale, Curr. Top. Med. Chem. 2001, 1, 151-159). Therefore, an individually dosage for anticoagulants of the class of selective factor Xa inhibitors is not required.

Inflammatory bowel diseases (IBD) encompass mainly the diseases Crohn's disease and Ulcerative colitis and a small number of patients with indeterminate colitis. They are chronic inflammatory diseases of the gastrointestinal tract. The inflammation in ulcerative colitis is mainly localized to the mucosa and submucosa, whereas in Crohn's disease the inflammation extends through the intestinal wall from mucosa to serosa. Ulcerative colitis is confined to the colon, and colectomy is therefore a curative procedure, although some patients may subsequently suffer from inflammation of the ileal pouch (pouchitis). Crohn's disease, in contrast, can involve any part of the gastrointestinal tract, although the distal end of the small bowel and the colon are most commonly involved. Hence, resection of the inflamed segment is not curative of Crohn's disease. The highest incidence of IBD occurs in Caucasian populations in northern Europe and North America, where the incidence for each disease is about 5 per 100,000 and the prevalence is approximately 50 per 100,000. The peak age at onset is between 15 and 25 years of age, with a second, lesser peak between 55 and 65 years, but it also occurs in childhood. A genetic cause is involved, as the most important risk factor for IBD is a positive family history. In IBD, the lamina propria is infiltrated with lymphocytes, macrophages, and other cells of the immune system. An intensive search for the antigens that trigger the immune response has yet to identify a specific microbial pathogen. Pathology: in ulcerative colitis, inflammation begins in the rectum, extends proximally a certain distance, and then abruptly stops, with a clear demarcation between involved and uninvolved mucosa. In mild disease, there are superficial erosions, whereas in more severe disease, ulcers may be large but superficial and penetrate the muscularis mucosa only in very severe disease. Most of the pathologic findings in ulcerative colitis are limited to the mucosa and submucosa; the muscularis propria is affected only in fulminant disease. In Crohn's disease, the bowel wall is thickened and stiff. The mesentery, which is thickened, edematous, and contracted, fixes the intestine in one position. Transmural inflammation may cause adhesions between adjacent loops of intestine. All layers of the intestine are thickened, and the lumen is narrowed. Clinical Symptoms: Ulcerative Colitis: diarrhea, usually associated with blood in the stool. Bowel movements are frequent but small in volume as a result of irritability of the inflamed rectum. Urgency and fecal incontinence may limit the patient's ability to function in society. Other symptoms include fever and pain, which may be in either lower quadrant or in the rectum. Fever, malaise, and weight loss can occur in severe cases and may have a greater effect than diarrhea on the patient's ability to function. Crohn's disease: the predominant symptoms are diarrhea, abdominal pain, and weight loss; any of these three symptoms may be most prominent in a given individual. Diarrhea occurs in almost all those with Crohn's disease. Crohn's disease, like ulcerative colitis, is a relapsing and remitting disease. About 30% of placebo-treated patients with Crohn's disease of mild to moderate activity go into remission within 4 months. Conversely, of patients in remission and not receiving therapy, about 30% relapse within 1 year and 50% at 2 years. Current treatment: Antidiarrheal agents (e.g. loperamide or diphenoxylate), Anticholinergics (e.g. tincture of belladonna, clidinium, propantheline bromide, and dicyclomine hydrochloride), combination of an antidiarrheal and an antispasmodic (e.g. powdered opium and belladonna). The chronic use of narcotics for pain should not be part of the management of IBD. Sometimes antidepressants can be helpful. Nonsteroidal anti-inflammatory drugs can exacerbate the clinical activity of IBD and should be used cautiously. Nutritional management plays only a small role in ulcerative colitis. Patients should avoid specific foods that worsen their symptoms, typically high-fiber foods. Agents that contain 5-aminosalicylic acid (5-ASA) (e.g. sulfasalazine, olsalazine, and balsalazide, Asacol and Pentasa) are mainstays of therapy for ulcerative colitis and play a small role in the management of Crohn's disease. Systemic or non-systemic corticosteroids (e.g. prednisone, budesonide), administered orally or rectally, are effective in mild to moderate ulcerative colitis and Crohn's disease. Immunomodulator drugs act by blocking lymphocyte proliferation, activation, or effector mechanisms, e.g. azathioprine and 6-mercaptopurine (6-MP), cyclosporine and methotrexate. Azathioprine and 6-MP are effective in treating active Crohn's disease and in maintaining remission; their role in ulcerative colitis is less clear. Antibiotics only in specific cases. Anti-Tumor Necrosis Factor Antibody (e.g. Infliximab).

Pathophysiological features of IBD encompass procoagulant blood state predisposing to thromboembolism [Lam, Gastroenterology 1975, 68, 245-251]. However, a clinical study has failed to show a clinical benefit for unfractionated heparin in ulcerative colits [Panes, Gastroenterology 2000, 4, 903-8]. In contrast, experimental and clinical studies have shown beneficial effects of low dose low-molecular-weight-heparin in concentrations that were not anticoagulative, suggesting drug effects distinct from anticoagulation to provide the benefit [Dotan, Alimen Pharmacol Ther 2001, 15, 1687-1697; Dotan, Digestive Diseases and Sciences 2001, 46, 2239-2244].

Celiac disease, which is caused by intolerance to gluten, is an inflammatory autoimmune disease affecting the upper small intestine (duodenum and jejunum) and resulting in flattening of the villi. Celiac disease is associated with malabsorption of nutrients, minerals and fat-soluble vitamins and the symptoms include diarrhea, intestinal bloating, abdominal pain weight loss, anemia and fatigue. The main treatment is strict adherence to a life-long gluten-free diet, and continued exposure to even small amounts of gluten can increase the risk of complications associated with celiac disease. Celiac disease has also been found to be associated with an increased risk of thromboembolic events [Ludvigsson Br J Haematol 2007, 139, 121-127].

In addition to their important role in the activation of the coagulation system leading to hypercoagulability and thrombosis, Factor Xa and thrombin are known to exhibit variant pleiotropic effects. Thus, they are potent mitogens that induce proliferation. They induce and/or augment vasoconstriction. Signaling by thrombin leads to proinflammatory cytokine release. Treatment with Factor Xa inhibitors, besides their inhibition of blood coagulation, may suppress the mitogenic, vasoconstrictive and inflammatory stimuli of Factor Xa and thrombin, the latter by inhibition of the generation of thrombin.

It has now been found, surprisingly, that selective inhibitors of coagulation factor Xa, in particular of oxazolidinones of the formula (I), are also suitable for the treatment and/or prophylaxis of inflammatory conditions of the gastrointestinal tract such as inflammatory bowel disease (IBD) and celiac disease and/or disorders related to inflammatory bowel disease (IBD) and/or celiac disease.

The present invention therefore relates to the use of selective factor Xa inhibitors for preparing medicaments or pharmaceutical compositions for the treatment and/or prophylaxis of inflammatory conditions of the gastrointestinal tract such as inflammatory bowel disease (IBD) and celiac disease and/or disorders related to inflammatory bowel disease (IBD) and/or celiac disease.

The present invention therefore relates in particular to the use of compounds of the formula (I) 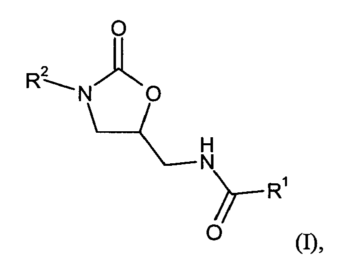 in which
- R¹: is 2-thiophene which is substituted in position 5 by a radical from the group of chlorine, bromine, methyl or trifluoromethyl,
- R²: is D-A-:
where:
the radical "A" is phenylene;
where:
the group "A" defined above may optionally be substituted once or twice in the meta position relative to the linkage to the oxazolidinone by a radical from the group of fluorine, chlorine, nitro, amino, trifluoromethyl, methyl or cyano,
the radical "D" is a saturated 5- or 6-membered heterocycle which is linked via a nitrogen atom to "A", which has a carbonyl group in direct vicinity to the linking nitrogen atom, and in which a ring carbon member may be replaced by a heteroatom from the series S, N and O;
and their pharmaceutically acceptable salts, solvates and solvates of the salts for preparing medicaments or pharmaceutical compositions for the treatment and/or prophylaxis of inflammatory conditions of the gastrointestinal tract such as inflammatory bowel disease (IBD) and celiac disease and/or disorders related to inflammatory bowel disease (IBD) and/or celiac disease.
Very particular preference is likewise given in this connection to the use of the compound 5-chloro-*N*-({(5*S*)-2-oxo-3-[4-(3-oxo-4-morpholinyl)phenyl]-1,3-oxazolidin-5-yl}methyl)-2-thiophenecarboxamide (rivaroxaban; example 1) having the following formula 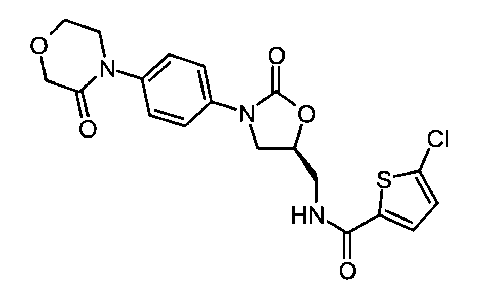 and its pharmaceutically acceptable salts, solvates and solvates of the salts for preparing medicaments or pharmaceutical compositions for the treatment and/or prophylaxis of inflammatory conditions of the gastrointestinal tract such as inflammatory bowel disease (IBD) and celiac disease and/or disorders related to inflammatory bowel disease (IBD) and/or celiac disease.

To date, oxazolidinones have been described essentially only as antibiotics, and in a few cases also as MAO inhibitors and fibrinogen antagonists (Review: B. Riedl, R. Endermann, Exp. Opin. Ther. Patents 1999, 9, 625), and a small 5-[acylaminomethyl] group (preferably 5-[acetylaminomethyl]) appears to be essential for the antibacterial effect.

Substituted aryl- and heteroarylphenyloxazolidinones in which a monosubstituted or polysubstituted phenyl radical may be bonded to the N atom of the oxazolidinone ring and which may have in position 5 of the oxazolidinone ring an unsubstituted N-methyl-2-thiophenecarboxamide residue, and their use as substances with antibacterial activity are disclosed in the U.S. patents US-A-5 929 248, US-A-5 801 246, US-A-5 756 732, US-A-5 654 435, US-A-5 654 428 and US-A-5 565 571.

In addition, benzamidine-containing oxazolidinones are known as synthetic intermediates in the synthesis of factor Xa inhibitors or fibrinogen antagonists (WO-A-99/31092, EP-A-623615).

Compounds according to the invention are the compounds of the formula (I) and salts, solvates and solvates of the salts thereof, the compounds of the formulae mentioned below covered by formula (I) and salts, solvates and solvates of the salts thereof and the compounds mentioned below as practical examples covered by formula (I) and salts, solvates and solvates of the salts thereof, insofar as the compounds of the formulae mentioned below covered by formula (I) are not already salts, solvates and solvates of the salts.

Depending on their structure, the compounds according to the invention can exist in stereo-isomeric forms (enantiomers, diastereomers). The present invention therefore includes the enantiomers or diastereomers and respective mixtures thereof. From such mixtures of enantiomers and/or diastereomers, the stereoisomerically homogeneous components can be isolated in known manner.

Insofar as the compounds according to the invention can occur in tautomeric forms, the present invention includes all tautomeric forms.

As salts in the context of the present invention, physiologically harmless salts of the compounds according to the invention are preferred. Also included are salts which are not themselves suitable for pharmaceutical applications, but can for example be used for the isolation or purification of the compounds according to the invention.

Physiologically harmless salts of the compounds according to the invention include acid addition salts of mineral acids, carboxylic acids and sulphonic acids, e.g. salts of hydrochloric acid, hydrobromic acid, sulphuric acid, phosphoric acid, methanesulphonic acid, ethane-sulphonic acid, toluene-sulphonic acid, benzenesulphonic acid, naphthalen-edisulphonic acid, acetic acid, trifluoroacetic acid, propionic acid, lactic acid, tartaric acid, malic acid, citric acid, fumaric acid, maleic acid and benzoic acid.

Physiologically harmless salts of the compounds according to the invention also include salts of usual bases, such as for example and preferably alkali metal salts (e.g. sodium and potassium salts), alkaline earth salts (e.g. calcium and magnesium salts) and ammonium salts, derived from ammonia or organic amines with 1 to 16 C atoms, such as for example and preferably ethylamine, diethylamine, triethylamine, ethyl-diiso-propyl-amine, monoethanolamine, diethanolamine, triethanolamine, dicyclohexylamine, dimethylaminoethanol, procaine, dibenzylamine, *N-*methyl-morpholine, arginine, lysine, ethylenediamine and *N*-methylpiperidine.

In the context of the invention those forms of the compounds according to the invention which in the solid or liquid state form a complex by coordination with solvent molecules are described as solvates. Hydrates are a specific form of solvates, wherein the coordination takes place with water. Hydrates are preferred as solvates in the context of the present invention.

In addition, the present invention also includes prodrugs of the compounds according to the invention. The term "prodrugs" includes compounds which can themselves be biologically active or inactive, but are converted into compounds according to the invention (for example metabolically or hydrolytically) during their residence time in the body.

In the context of the present invention, unless otherwise specified, the substituents have the following meaning:

A saturated 5- or 6-membered heterocycle which is linked via a nitrogen atom to "A", which has a carbonyl group in direct vicinity to the linking nitrogen atom, and in which a ring carbon member may be replaced by a heteroatom from the series S, N and O, may be mentioned for example as: 2-oxo-pyrrolidine-1-yl, 2-oxo-piperidine-1-yl, 2-oxo-piperazine-1-yl, 2-oxo-morpholine-1-yl, 3-oxo-thiomorpholine-4-yl, 2-oxo-1,3-oxazolidine-1-yl, 2-oxo-1,3-oxazinan-1-yl, 2-oxo-imidazolidine-1-yl und 2-oxo-tetrahydropyrimidine-1-yl.

The compounds of the formula (1) can be prepared by either, in a process alternative,
[A] reacting compounds of the general formula (II) 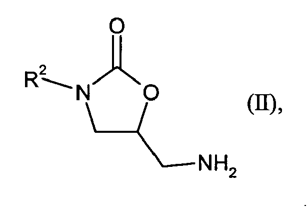 in which
   the radical R² has the meaning indicated above,
   with carboxylic acids of the general formula (III) 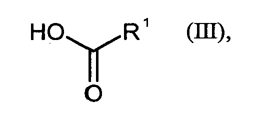 in which
   the radical R¹ has the meaning indicated above,
   or else with the corresponding carbonyl halides, preferably carbonyl chlorides, or else with the corresponding symmetrical or mixed carboxylic anhydrides of the carboxylic acids of the general formula (III) defined above
   in inert solvents, where appropriate in the presence of an activating or coupling reagent
   and/or of a base, to give compounds of the general formula (I)
   or else in a process alternative
[B] converting compounds of the general formula (IV) 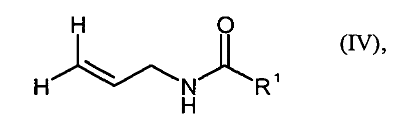 in which
   the radical R¹ has the meanings indicated above,
   with a suitable selective oxidizing agent in an inert solvent into the corresponding epoxide of the general formula (V) 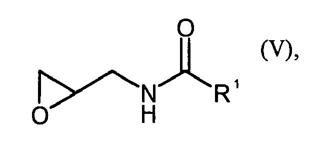 in which
   the radical R¹ has the meanings indicated above,
   and are reacted in an inert solvent, where appropriate in the presence of a catalyst, with an amine of the general formula (VI)

   R²-NH₂ (VI),

   in which
   the radical R² has the meaning indicated above,
   initially preparing the compounds of the general formula (VII) 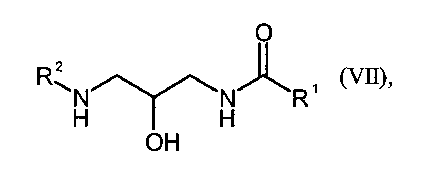 in which
   the radicals R¹ and R² have the meanings indicated above,
   and
   subsequently cyclizing in an inert solvent in the presence of phosgene or phosgene equivalents such as, for example, carbonyldiimidazole (CDI) to the compounds of the general formula (I).

Solvents suitable for the processes described above are in these cases organic solvents which are inert under the reaction conditions. These include halohydrocarbons such as dichloromethane, trichloromethane, tetrachloromethane, 1,2-dichloroethane, trichloroethane, tetrachloroethane, 1,2-dichloroethylene or trichloroethylene, ethers such as diethyl ether, dioxane, tetrahydrofuran, glycol dimethyl ether or diethylene glycol dimethyl ether, alcohols such as methanol, ethanol, n-propanol, isopropanol, n-butanol or tert-butanol, hydrocarbons such as benzene, xylene, toluene, hexane or cyclohexane, dimethylformamide, dimethyl sulfoxide, acetonitrile, pyridine, hexamethylphosphoric triamide or water.

It is likewise possible to employ solvent mixtures composed of the aforementioned solvents.

Activating or coupling reagents suitable for the processes described above are in these cases the reagents normally used for these purposes, for example *N'*-(3-dimethylaminopropyl)-*N-*ethylcarbodiimide •HCl, *N,N'*-dicyclohexylcarbodiimide, 1-hydroxy-1H-benzotriazole • H₂O and the like.

Suitable bases are the usual inorganic or organic bases. These preferably include alkali metal hydroxides such as, for example, sodium or potassium hydroxide or alkali metal carbonates such as sodium or potassium carbonate or sodium or potassium methanolate or sodium or potassium ethanolate or potassium tert-butoxide or amides such as sodamide, lithium bis-(trimethylsilyl)amide or lithium diisopropylamide or amines such as triethylamine, diisopropylethylamine, diisopropylamine, 4-*N,N*-dimethylaminopyridine or pyridine.

The base can be employed in these cases in an amount of from 1 to 5 mol, preferably from 1 to 2 mol, based on 1 mol of the compounds of the general formula (II).

The reactions generally take place in a temperature range from -78°C to the reflux temperature, preferably in the range from 0°C to the reflux temperature.

The reactions can be carried out under atmospheric, elevated or reduced pressure (e.g. in the range from 0.5 to 5 bar), generally under atmospheric pressure.

Suitable selective oxidizing agents both the preparing epoxides and for the oxidation which is optionally carred out to the sulfone, sulfoxide or N-oxide are, for example, m-chloroperbenzoic acid (MCPBA), sodium metaperiodate, N-methylmorpholine N-oxide (NMO), monoperoxyphthalic acid or osmium tetroxide.

The conditions used for preparing the epoxides are those customary for these preparations.

For detailed conditions for the process of oxidation, which is carried out where appropriate, to the sulfone, sulfoxide or N-oxide, reference may be made to the following literature: M.R. Barbachyn et al. J. Med. Chem. 1996, 39, 680 and WO-A-97/10223.

The compounds of the formulae (II), (III), (IV) and (VI) are known per se to the skilled worker or can be prepared by conventional methods. For oxazolidinones, in particular the 5-(aminomethyl)-2-oxooxazolidines required, cf. WO-A-98/01446; WO-A-93/23384; WO-A-97/03072; J.A. Tucker et al. J. Med. Chem. 1998, 41, 3727; S.J. Brickner et al. J. Med. Chem. 1996, 39, 673; W.A. Gregory et al. J. Med. Chem. 1989, 32, 1673.

The process for the synthesis of the compounds of the general formula (I) is described in detail in WO-A-01/047919.

For the purpose of the present invention "inflammatory bowel disease (IBD)" include, in particular, serious disorders such as Crohn's disease, Ulcerative colitis and chronic inflammatory diseases of the gastrointestinal tract.

For the purpose of the present invention "disorders related to inflammatory bowel disease (IBD) and/or celiac disease" include, in particular, major arterial and venous thromboembolic events, pulmonary embolism (PE), myocardial infarction and stroke.

Furthermore, the present invention relates to a method for the treatment and/or prophylaxis of inflammatory conditions of the gastrointestinal tract such as inflammatory bowel disease (IBD) and celiac disease and/or disorders related to inflammatory bowel disease (IBD) and/or celiac disease of the human or animal body with the use of an effective quantity of a selective factor Xa inhibitor or of a medicament, comprising at least one selective factor Xa inhibitor in combination with one or more pharmacologically acceptable auxiliaries or excipients.

Furthermore, the present invention relates to a method for the treatment and/or prophylaxis of inflammatory conditions of the gastrointestinal tract such as inflammatory bowel disease (IBD) and celiac disease and/or disorders related to inflammatory bowel disease (IBD) and/or celiac disease of the human or animal body with the use of an effective quantity of at least one compound of the formula (I) or of a medicament, comprising at least one compound of the formula (I) in combination with one or more pharmacologically acceptable auxiliaries or excipients.

Furthermore, the present invention relates to a method for the treatment and/or prophylaxis of inflammatory conditions of the gastrointestinal tract such as inflammatory bowel disease (IBD) and celiac disease and/or disorders related to inflammatory bowel disease (IBD) and/or celiac disease of the human or animal body with the use of an effective quantity of at least the compound 5-chloro-*N-*({(5*S*)-2-oxo-3-[4-(3-oxo-4-morpholinyl)phenyl]-1,3-oxazolidin-5-yl}methyl)-2-thiophenecarboxamide or of a medicament, comprising at least the compound 5-chloro-*N*-({(5*S*)-2-oxo-3-[4-(3-oxo-4-morpholinyl)phenyl]-1,3-oxazolidin-5-yl}methyl)-2-thiophenecarbox-amide in combination with one or more pharmacologically acceptable auxiliaries or excipients.

The present invention further provides medicaments and pharmaceutical compositions comprising at least one compound of the formula (I) according to the invention together with one or more pharmacologically acceptable auxiliaries or excipients, which medicaments and pharmaceutical compositions can be used for the indications mentioned above.

In one embodiment, the invention provides a method for preventing the formation of thrombi in the microvasculature and macrovasculature, wherein the method comprises administering on a chronic basis to the mammal a therapeutically effective amount of at least one compound of the formula (I) such as rivaroxaban.

In another embodiment, the method provides for the improved survival that occur in the mammal, the method comprising the steps of administering on a chronic basis to the mammal a therapeutically effective amount of at least one compound of the formula (I) such as rivaroxaban, wherein the previously specified events' frequency are reduced relative to the frequency established by a recognized standard of care.

In another embodiment, the method provides for a reduction of the number of hospitalizations required for the care of the individual, the method comprising the steps of administering on a chronic basis to the mammal a therapeutically effective amount of at least one compound of the formula (I) such as rivaroxaban, wherein the previously specified event's frequency is reduced relative to the frequency established by a recognized standard of care.

In another embodiment, the method provides for a replacement therapy in the mammal for other anticoagulant and antiplatelet therapies that represent current guideline based standards of care, the method comprising the steps of administering on a chronic basis to the mammal a therapeutically effective amount of at least one compound of the formula (I) such as rivaroxaban. The frequency of events that are observed in the mammal are equal to or reduced relative to the frequency established by the recognized standard of care which is being replaced.

In another embodiment, the present invention also relates to the combinations of
A) compounds of the formula (I) together with
B) other pharmaceutical drugs, especially with antidiarrheal agents, anticholinergic agents, a combination of an antidiarrheal agent and an antispasmodic agent, pain relievers, antidepressants, nonsteroidal anti-inflammatory drugs, agents that contain 5-aminosalicylic acid (5-ASA), oral corticosteroids, immunomodulator drugs, antibiotics and/or an anti-Tumor Necrosis Factor Antibody.

"Combinations" in the context of the present application are not only pharmaceutical formulations, which comprise all components (so-called fixed-dose combinations), and combination packages, which keep all components separate from each other, but also components which are administered simultaneously or temporary staggered, provided that they are used for the prophylaxis and/or treatment of the same disease. In addition, it is possible to combine two or more drugs together, which are dual or multiple combinations, respectively.

The individual drugs of the combinations are known from literature and are mostly commercially available.

Antidiarrheal agents are for example loperamide and diphenoxylate.

Anticholinergic agents are for example tincture of belladonna, clidinium, propantheline bromide, and dicyclomine hydrochloride.

Combination treatments of an antidiarrheal and an antispasmodic agent are for example powdered opium and belladonna.

Pain relievers or nonsteroidal anti-inflammatory drugs (NSAID) are for example salicylates, for example acetylsalicylic acid (Aspirin), benorylate/benorilate, choline magnesium salicylate, diflunisal, ethenzamide, faislamine, methyl salicylate, magnesium salicylate, salicyl salicylate or salicylamide; arylalkanoic acid for example diclofenac, aceclofenac, acemetacin, alclofenac, bromfenac, etodolac, indometacin, nabumetone, oxametacin, proglumetacin, sulindac or tolmetin; 2-arylpropionic acid for example ibuprofen, alminoprofen, carprofen, dexibuprofen, dexketoprofen, fenbufen, fenoprofen, flunoxaprofen, flurbiprofen, ibuproxam, indoprofen, ketoprofen, ketorolac, loxoprofen, naproxen, oxaprozin, pirprofen, suprofen or tiaprofenic acid; *N-*arylanthranilic acids (fenamic acids) for example mefenamic acid, flufenamic acid, meclofenamic acid or tolfenamic acid; pyrazolidine derivates, for example phenylbutazone, ampyrone, azapropazone, clofezone, kebuzone, metamizole, mofebutazone, oxyphenbutazone, phenazone, phenylbutazone or sulfinpyrazone; oxicams for example piroxicam, droxicam, lornoxicam, meloxicam or tenoxicam and COX-2- inhibitors for example celecoxib.

Antidepressants are for example mirtazapine, bupropion, paroxetine, selective serotonin reuptake inhibitor or selective norepinephrine serotonin reuptake inhibitor.

Agents that contain 5-aminosalicylic acid (5-ASA) are for example sulfasalazine, olsalazine, and balsalazide, Asacol and Pentasa.

Oral corticosteroids are for example prednisone.

Immunomodulator drugs are for example azathioprine and 6-mercaptopurine (6-MP), cyclosporine and methotrexate.

Antibiotics are for example aminoglycosides for example amikacin, gentamicin, kanamycin, neomycin, streptomycin, tobramycin or paromomycin; ansamycins for example geldanamycin or herbimycin; carbacephem for example loracarbef; carbapenems for example ertapenem, doripenem, imipenem/cilastatin, meropenem; cephalosporins (first generation) for example cefadroxil, cefazolin, cefalotin or cefalothin, cefalexin; cephalosporins (second generation) for example cefaclor, cefamandole, cefoxitin, cefprozil or cefuroxime; cephalosporins (third generation) for example cefixime, cefdinir, cefditoren, cefoperazone, cefotaxime, cefpodoxime, ceftazidime, ceftibuten, ceftizoxime or ceftriaxone; cephalosporins (fourth generation) for example cefepime; cephalosporins (fifth generation) for example ceftobiprole; glycopeptides for example teicoplanin or vancomycin; macrolides for example azithromycin, clarithromycin, dirithromycin, erythromycin, roxithromycin, troleandomycin, telithromycin or spectinomycin; monobactams for example aztreonam; penicillins for example amoxicillin, ampicillin, azlocillin, carbenicillin, cloxacillin, dicloxacillin, flucloxacillin, mezlocillin, meticillin, nafcillin, oxacillin, penicillin, piperacillin or ticarcillin; polypeptides for example bacitracin, colistin or polymyxin B; quinolones for example ciprofloxacin, enoxacin, gatifloxacin, levofloxacin, lomefloxacin, moxifloxacin, norfloxacin, ofloxacin or trovafloxacin; sulfonamides for example mafenide, prontosil (archaic), sulfacetamide, sulfamethizole, sulfanilimide (archaic), sulfasalazine, sulfisoxazole, trimethoprim or trimethoprim-sulfamethoxazole (co-trimoxazole) (TMP-SMX); tetracyclines for example demeclocycline, doxycycline, minocycline, oxytetracycline or tetracycline; other antibiotics for example arsphenamine, chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin/dalfopristin, rifampin, rifampicin or tinidazole.

Anti-Tumor Necrosis Factor Antibody is for example Infliximab.

Furthermore, the present invention relates to drugs which comprise at least one compound according to the invention, together with one or more inert, non-toxic and pharmaceutically appropriate adjuvants, as well as their use for the above-mentioned purposes.

Furthermore, the present invention relates to drugs which comprise at least one compound according to the invention, together with one or more of the above-mentioned combination drug, especially for the use for the prophylaxis and/or treatment of the above-mentioned diseases.

The compounds according to the invention can act systemically and/or locally. For this purpose, they can be administered in a suitable manner, such as for example by the oral, parenteral, pulmonary, nasal, sublingual, lingual, buccal, rectal, dermal, transdermal, conjunctival or aural routes or as an implant or stent.

For these administration routes, the compounds according to the invention can be administered in suitable administration forms.

For oral administration, administration forms which function according to the state of the art, releasing the compound according to the invention rapidly and/or in a modified manner, which contain the compounds according to the invention in crystalline and/or amorphized and/or dissolved form, such as for example tablets (uncoated or coated tablets, for example with gastric juice-resistant or delayed dissolution or insoluble coatings, which control the release of the compound according to the invention), tablets rapidly disintegrating in the oral cavity or films/wafers, films/lyophilisates, capsules (for example hard or soft gelatine capsules), dragees, granules, pellets, powders, emulsions, suspensions, aerosols or solutions are suitable.

Parenteral administration can be effected omitting an absorption step (e.g. intravenous, intraarterial, intracardial, intraspinal or intralumbar administration) or involving absorption (e.g. intramuscular, subcutaneous, intracutaneous, percutaneous or intraperitoneal administration). Suitable administration forms for parenteral administration include injection and infusion preparations in the form of solutions, suspensions, emulsions, lyophilisates or sterile powders.

For the other administration routes, for example inhalation formulations (including powder inhalers and nebulisers), nasal drops, solutions or sprays, tablets for lingual, sublingual or buccal administration, tablets, films/wafers or capsules, suppositories, oral or ophthalmic preparations, vaginal capsules, aqueous suspensions (lotions, shakable mixtures), lipophilic suspensions, ointments, creams, transdermal therapeutic systems (e.g. plasters), milk, pastes, foams, dusting powders, implants or stents are suitable.

Oral or parenteral administration, in particular oral and intravenous administration, are preferred.

The compounds according to the invention can be converted into the stated administration forms. This can be effected in a manner known per se by mixing with inert, non-toxic, pharmaceutically suitable additives. These additives include carriers (for example microcrystalline cellulose, lactose or mannitol), solvents (e.g. liquid polyethylene glycols), emulsifiers and dispersants or wetting agents (for example sodium dodecylsulphate, polyoxysorbitan oleate), binders (for example polyvinylpyrrolidone), synthetic and natural polymers (for example albumin), stabilizers (e.g. antioxidants such as for example ascorbic acid), colourants (e.g. inorganic pigments such as for example iron oxide) and flavour or odour correctors.

In general, to achieve effective results in parenteral administration it has been found advantageous to administer quantities of about 0.001 to 30 mg/kg, preferably about 0.01 to 1 mg/kg body weight. In oral administration, the dosage is about 0.01 bis 100 mg/kg, preferably about 0.01 to 30 mg/kg and quite especially preferably 1 to 30 mg/kg body weight.

Nonetheless it can sometimes be necessary to deviate from the said quantities, namely depending on body weight, administration route, individual response to the active substance, nature of the preparation and time or interval at which administration takes place. Thus in some cases it can be sufficient to manage with less than the aforesaid minimum quantity, while in other cases the stated upper limit must be exceeded. In the event of administration of larger quantities, it may be advisable to divide these into several individual administrations through the day.

The present invention is illustrated by the examples below; however, these examples are not meant to restrict the invention in any way.

The percentages in the tests and examples which follows are, unless otherwise stated, by weight; parts are by weight. Solvent ratios, dilution ratios and concentrations reported for liquid/liquid solutions are each based on the volume.

### Examples

### A Preparation examples

### Starting materials

The syntheses of the starting materials are described in detail in WO-A-01/047919.

### Synthesis examples

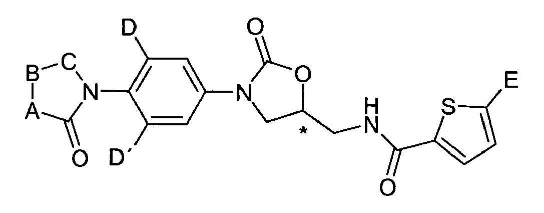

| **Example** | **A-B-C** | **D** | **D'** | **E** |
|---|---|---|---|---|
| 1 | CH₂OCH₂CH₂ | H | H | Cl |
| 2 | CH₂CH₂CH₂ | H | H | Cl |
| 3 | CH₂CH₂CH₂ | H | H | CH₃ |
| 4 | CH₂CH₂CH₂ | H | H | Br |
| 5 | CH₂OCH₂CH₂ | H | H | CH₃ |
| 6 | CH₂OCH₂CH₂ | H | H | Br |
| 7 | OCH₂CH₂ | H | H | Cl |
| 8 | CH₂CH₂CH₂ | H | H | Br |
| 9 | CH₂CH₂CH₂ | H | H | CH₃ |
| 10 | OCH₂CH₂CH₂ | H | H | Cl |
| 11 | CH₂CH₂CH₂ | F | H | Cl |
| 12 | CH₂OCH₂CH₂ | H | H | Cl |
| 13 | CH₂CH₂CH₂ | CF₃ | H | Cl |
| 14 | CH₂OCH₂CH₂ | Cl | H | Cl |
| 15 | CH₂OCH₂CH₂ | CF₃ | H | Cl |
| 16 | CH₂OCH₂CH₂ | CH₃ | H | Cl |
| 17 | CH₂OCH₂CH₂ | CN | H | Cl |
| 18 | CH₂CH₂CH₂ | Cl | H | Cl |
| 19 | CH₂OCH₂CH₂ | CH₃ | CH₃ | Cl |
| 20 | CH₂OCH₂CH₂ | NH₂ | H | Cl |
| 21 | CH₂OCH₂CH₂ | F | H | Br |
| 22 | CH₂CH₂CH₂ | F | H | Br |
| 23 | CH₂CH₂CH₂CH₂ | H | H | Br |
| 24 | CH₂CH₂CH₂ | F | H | Cl |
| 25 | CH₂OCH₂CH₂ | F | H | Cl |
| 26 | CH₂CH₂CH₂CH₂ | H | H | Cl |

The syntheses of the synthesis examples are described in detail in WO-A-01/047919.

### B Assessment of the physiological activity

### 1. Physiological activity of compounds of the formula (I)

The compounds of the formula (I) act in particular as selective inhibitors of coagulation factor Xa and do not inhibit, or also inhibit only at distinctly higher concentrations, other serine proteases such as plasmin or trypsin.

Inhibitors of coagulation factor Xa are referred to as "selective" when their IC₅₀ values for factor Xa inhibition are 100-fold, preferably 500-fold, in particular 1000-fold, smaller than the IC₅₀ values for the inhibition of other serine proteases, in particular plasmin and trypsin, reference being made concerning the test methods for the selectivity to the test methods of Examples A.a.1) and A.a.2) described below.

The particularly advantageous biological properties of the compounds of the formula (I) can be ascertained by the following methods.

### a) Test description (in vitro)

### a.1) Measurement of factor Xa inhibition

The enzymatic activity of human factor Xa (FXa) is measured via the conversion of an FXa-specific chromogenic substrate. In this case, factor Xa eliminates p-nitroaniline from the chromogenic substrate. The determinations are carried out in microtiter plates as follows.

The test substances are dissolved in various concentrations in DMSO and incubated with human FXa (0.5 nmol/l dissolved in 50 mmol/l tris buffer [C,C,C-tris(hydroxymethyl)-aminomethane], 150 mmol/l NaCl, 0.1% BSA (bovine serum albumine), pH = 8.3) at 25°C for 10 minutes. Pure DMSO serves as control. The chromogenic substrate (150 µmol/l Pefachrome^{®} FXa from Pentapharm) is then added. After incubation at 25°C for 20 minutes, the extinction at 405 nm is determined. The extinctions of the test mixtures with test substance are compared with the control mixtures without test substance, and the IC₅₀ values are calculated therefrom.

### a.2) Selectivity determination

Selective FXa inhibition is demonstrated by investingating the inhibition by the test substances of other human serine proteases such as trypsin, plasmin. The enzymatic activity of trypsin (500 mU/ml) and plasmin (3.2 nmol/l) is determined by dissolving these enzymes in tris buffer (100 mmol/l, 20 mmol/l CaCl₂, pH = 8.0) and incubating with test substance or solvent for 10 minutes. The enzymatic reaction is then started by adding the appropriate specific chromogenic substrates (Chromozym Trypsin^{®} from Boehringer Mannheim, Chromozym Plasmin^{®} from Boehringer Mannheim), and the extinction is determined at 405 nm after 20 minutes. All determinations are carried out at 37°C. The extinctions of the test mixtures with test substance are compared with the control samples without test substance, and the IC₅₀ values are calculated therefrom.

### a.3) Determination of the anticoagulant effect

The anticoagulant effect of the test substances is determined in vitro in human plasma. For this purpose, human blood is collected in a 0.11 molar sodium citrate solution in the sodium citrate/blood mixing ratio of 1/9. The blood is thoroughly mixed after collection and centrifuged at about 2000 g for 10 minutes. The supernatant is removed by pipette. The prothrombin time (PT, synonym: Quick's test) is determined in the presence of varying concentrations of test substance or the appropriate solvent using a commercially available test kit (Neoplastin^{®} from Boehringer Mannheim). The test compounds are incubated with the plasma at 37°C for 10 minutes. Coagulation is then induced by adding thromboplastin, and the time of onset of coagulation is determined. The concentration of test substance which brings about a doubling of the prothrombin time is found.

### b) Determination of the antithrombotic effect (in vivo)

### b.1) Arteriovenous shunt model (rat)

Fasting male rats (strain: HSD CPB:WU) weighing 200-250 g are anesthetized with a Rompun/Ketavet solution (12 mg/kg/50 mg/kg). Thrombus formation is induced in an arteriovenous shunt by a method based on that described by Christopher N. Berry et al., Br. J. Pharmacol. (1994), 113, 1209-1214. For this purpose, the left jugular vein and the right carotid artery are exposed. An extracorporeal shunt is formed between the two vessels using a 10 cm-long polyethylene tube (PE 60). This polyethylene tube is secured in the middle by tying in a further 3 cm-long polyethylene tube (PE 160) which contained a roughened nylon thread forming a loop to produce a thrombogenic surface. The extracorporeal circulation is maintained for 15 minutes. The shunt is then removed and the nylon thread with the thrombus is immediately weighed. The blank weight of the nylon thread has been found before the start of the experiment. The test substances are administered either intravenously through the tail vein or orally by gavage to conscious animals before setting up the extracorporeal circulation.

### b.2) Models of hapten-induced inflammatory bowel disease in mice and rats

Mice or rats are treated by intrarectal administration of dinitrobenzene sulphonic acid (DNBS) in ethanol, e.g. 0.25 ml of 50% ethanol containing 30 mg of DNBS [Dotan, Alimen Pharmacol Ther 2001, 15, 1687-1697]. In a variation of the model, in mice or rats, administration of the hapten 2,4,6-trinitrobenzenesulfonic acid (5-30 mg) in 0.25 ml of 50% ethanol as the "barrier breaker" produce dose-dependent colonic ulceration and inflammation. [Morris, Gastroenterology 1989, 96, 795-803]. Furthermore methods analog to Neurath, J Exp Med. 1995, 182(5), 1281-90; Dohi, J Exp Med. 1999, 189(8), 1169-80; Dohi, Gastroenterology 2000, 119(3), 724-33; Heller, Immunity 2002, 17(5), 629-38 and Nieuwenhuis, Proc Natl Acad Sci U S A. 2002, 99(26), 16951-6 can be used. In a further model, mice or rats are treated with the hapten oxazolone to induce colonic inflammation [Kojima, J Pharmacol Sci 2004, 96, 307-313; Ekström, Scand J Gastroenterol 1998, 33, 174-179; Boivirant, J Exp Med 1998, 188, 1929-1939].

### b.3) Model of Iodoacetamide (IA) -induced inflammatory bowel disease in mice and rats

Mice or rats are treated by intrarectal administration of Iodoacetamide (IA), for example in rats with 0.1 ml IA 3% [Dotan, Alimen Pharmacol Ther 2001, 15, 1687-1697; Rachmilewitz, Gastroenterology 1995, 109, 98-106; Reifen J, Gastroenterol 2004, 39, 514-9].

### b.4) Model of Dextran Sulfate Sodium (DSS)-induced inflammatory bowel disease in mice and rats

Mice or rats are treated for several days with Dextran Sulfate Sodium (DSS) polymers in drinking water to induce acute or chronic colitis [Okayasu, Gastroenterology 1990, 98(3), 694-702; Mahler, Am J Physiol. 1998, 274(3 Pt 1), G544-51].

### b.5) Genetic models of inflammatory bowel disease in mice and rats

Rats transgenic for human HLA-B27 and β2-microglobulin develop spontaneous inflammatory bowel disease [Sartor, Int Rev Immunol 2000, 19, 39-50]. Mice of the SAMP1/Yit strain [Kosiewicz, J Clin Invest 2001, 107, 695-702], as well as mice genetically manipulated to be deficient in e.g. IL-10 [Kuhn, Cell 1993, 75, 263-274] or mdrla [Panwala, J Immunol 1998, 161, 5733-5744] also develop spontaneous inflammatory lesions of the gastrointestinal tract.

### C Practical Examples of Pharmaceutical Compositions

The compounds according to the invention can be converted into pharmaceutical preparations as follows:

### Tablet:

### Composition:

100 mg of the compound according to the invention, 50 mg of lactose (monohydrate), 50 mg of maize starch (native), 10 mg of polyvinylpyrrolidone (PVP 25) (BASF Co., Ludwigshafen, Germany) and 2 mg of magnesium stearate.

Tablet weight 212 mg, diameter 8 mm, radius of curvature 12 mm.

### Production:

The mixture of compound according to the invention, lactose and starch is granulated with a 5% solution (w/w) of the PVP in water. After drying, the granulate is mixed with the magnesium stearate for 5 minutes. This mixture is compressed with a normal tablet press (tablet format: see above). As a guideline, a compression force of 15 kN is used for the compression.

### Orally Dosable Suspension:

### Composition:

1000 mg of the compound according to the invention, 1000 mg of ethanol (96%), 400 mg of Rhodigel^{®} (xanthan gum from FMC Co., Pennsylvania, USA) and 99 g water.

10 ml of oral suspension correspond to a single dose of 100 mg of the compound according to the invention.

### Production:

The Rhodigel is suspended in ethanol, and the compound according to the invention is added to the suspension. The water is added with stirring. The mixture is stirred for ca. 6 hrs until completion of the swelling of the Rhodigel.

### Orally dosable solution:

### Composition:

500 mg of the compound according to the invention, 2.5 g of polysorbate and 97 g of polyethylene glycol 400.20 g of oral solution correspond to a single dose of 100 mg of the compound according to the invention.

### Production:

The compound according to the invention is suspended with stirring in the mixture of polyethylene glycol and polysorbate. The stirring process is continued until the complete dissolution of the compound according to the invention.

### i.v. Solution:

The compound according to the invention is dissolved in a physiologically compatible solvent (e.g. isotonic sodium chloride solution, 5% glucose solution and/or 30% PEG 400 solution) at a concentration below the saturation solubility. The solution is sterile-filtered and filled into sterile and pyrogen-free injection containers.

## Claims

1. Use of a compound of the formula (I) 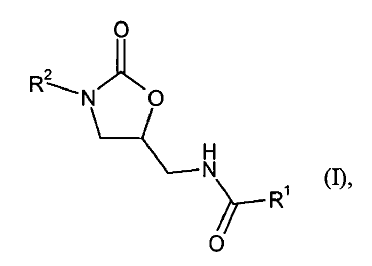 in which
R¹ is 2-thiophene which is substituted in position 5 by a radical from the group of chlorine, bromine, methyl or trifluoromethyl,
R² is D-A-:
where:
the radical "A" is phenylene;
where:
the group "A" defined above may optionally be substituted once or twice in the meta position relative to the linkage to the oxazolidinone by a radical from the group of fluorine, chlorine, nitro, amino, trifluoromethyl, methyl or cyano,
the radical "D" is a saturated 5- or 6-membered heterocycle which is linked via a nitrogen atom to "A", which has a carbonyl group in direct vicinity to the linking nitrogen atom, and in which a ring carbon member may be replaced by a heteroatom from the series S, N and O;
and their pharmaceutically acceptable salts, solvates and solvates of the salts for preparing medicaments or pharmaceutical compositions for the treatment and/or prophylaxis of inflammatory conditions of the gastrointestinal tract such as inflammatory bowel disease (IBD) and celiac disease and/or disorders related to inflammatory bowel disease (IBD) and/or celiac disease.

2. Use according to claim 1, **characterized in that** the compound of the formula (I) is 5-chloro-*N*-({(5*S*)-2-oxo-3-[4-(3-oxo-4-morpholinyl)phenyl]-1,3-oxazolidin-5-yl}methyl)-2-thiophenecarboxamide 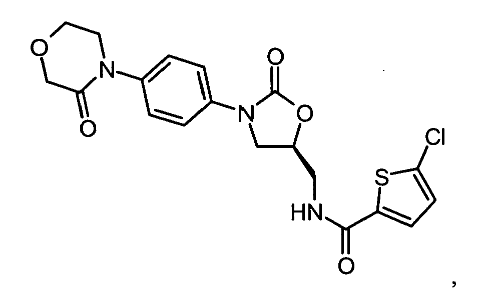 or its pharmaceutically acceptable salts, solvates and solvates of the salts.

3. Use of a compound of the formula (I) according to claim 1 or 2, **characterized in that** inflammatory bowel disease (IBD) is Crohn's disease, Ulcerative colitis and/or chronic inflammatory diseases of the gastrointestinal tract.

4. Use of a compound of the formula (I) according to claim 1 or 2, **characterized in that** disorders related to inflammatory bowel disease (IBD) and/or celiac disease are major arterial and venous thromboembolic events, pulmonary embolism (PE), myocardial infarction and/or stroke.

5. Method for the treatment and/or prophylaxis of inflammatory conditions of the gastrointestinal tract such as inflammatory bowel disease (IBD) and celiac disease and/or disorders related to inflammatory bowel disease (IBD) and/or celiac disease of the human or animal body with the use of an effective quantity of at least one compound of the formula (I) according to claim 1 or 2, or of a medicament, comprising at least one compound of the formula (I) according to claim 1 or 2, in combination with one or more pharmacologically acceptable auxiliaries or excipients

6. Method for the treatment and/or prophylaxis of inflammatory conditions of the gastrointestinal tract such as inflammatory bowel disease (IBD) and celiac disease and/or disorders related to inflammatory bowel disease (IBD) and/or celiac disease according to claim 5, **characterized in that** inflammatory bowel disease (IBD) is Crohn's disease, Ulcerative colitis and/or chronic inflammatory diseases of the gastrointestinal tract.

7. Method for the treatment and/or prophylaxis of inflammatory conditions of the gastrointestinal tract such as inflammatory bowel disease (IBD) and celiac disease and/or disorders related to inflammatory bowel disease (IBD) and/or celiac disease according to claim 5, **characterized in that** disorders related to inflammatory bowel disease (IBD) and/or celiac disease are major arterial and venous thromboembolic events, pulmonary embolism (PE), myocardial infarction and/or stroke.
